# EUROPÄISCHE PATENTSCHRIFT

(11) **EP 4 059 918 B1**
(45) Veröffentlichungstag und Bekanntmachung des Hinweises auf die Patenterteilung: **11.10.2023**
(21) Anmeldenummer: 21163482.9
(22) Anmeldetag: 18.03.2021
(51) Int. Cl.: C07F 9/6574, C07C 45/50

(54) **BISPHOSPHITLIGANDEN AUF BASIS VON BENZOPINAKOL MIT EINEM OFFENEN FLÜGELBAUSTEIN**
BISPHOSPHITE LIGANDS BASED ON BENZOPINACOL HAVING AN OPEN BUILDING BLOCK
LIGANDS BISPHOSPHITE À BASE DE BENZOPINACOL COMPORTANT UN COMPOSANT PÉRIPHÉRIQUE OUVERT

(43) Veröffentlichungstag der Anmeldung: 21.09.2022
(73) Patentinhaber: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); MARKOVIC, Ana, 45721 Haltern am See (DE); KUCMIERCZYK, Peter, 44628 Herne (DE); KNOSSALLA, Johannes, 46514 Gahlen (DE); SELENT, Detlef, 18059 Rostock (DE); BÖRNER, Armin, 18059 Rostock (DE); ROMEIKE, Kerstin, 18109 Rostock (DE)
(74) Vertreter: Evonik Patent Association

(56) Entgegenhaltungen:
- DE-A1-102006 058 682

## Beschreibung

Die vorliegende Erfindung betrifft Bisphosphitliganden auf Basis von Benzpinako mit einem offenen Flügelbaustein, sowie deren Verwendung in der Hydroformylierung.

In WO 2008/071508 A1 wird ein Verfahren zur Hydroformylierung unter Einsatz von Bisphosphitliganden beschrieben. Unter anderem wird der Einsatz des Liganden (D-1) beschrieben.

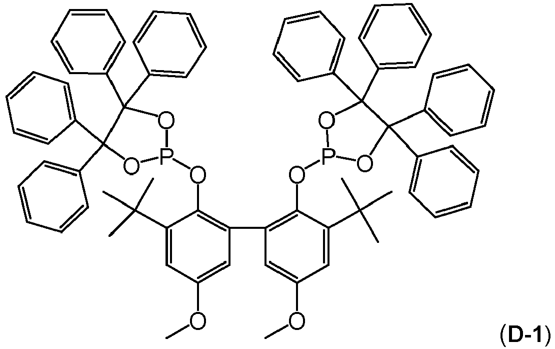

Die technische Aufgabe, welche der vorliegenden Erfindung zugrunde lag, ist die Bereitstellung neuer Verbindungen, welche gegenüber dem aus dem Stand der Technik bekannten Verbindungen bei der Hydroformylierung von Olefinen eine gesteigerte Ausbeute liefern.

Diese Aufgabe wird gelöst durch eine Verbindung nach Anspruch 1.

Verbindung gemäß Formel (I): 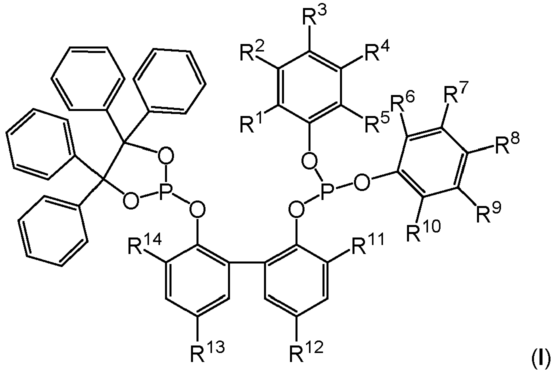 wobei
R¹, R², R³, R⁴, R⁵, R⁶, R⁷, R⁸, R⁹, R¹⁰, R¹¹, R¹², R¹³, R¹⁴ jeweils unabhängig voneinander ausgewählt sind aus: -H, -(C₁-C₁₂)-Alkyl, -O-(C₁-C₁₂)-Alkyl, -(C₄-C₁₂)-Aryl und die Reste R¹, R², R³, R⁴, R⁵, sowie die Reste R⁶, R⁷, R⁸, R⁹, R¹⁰ untereinander kondensierte Systeme ausbilden können.

Die Reste R¹, R², R³, R⁴, R⁵, können also über einen Ring oder mehrere Ringe miteinander verbunden sein und dadurch ein neues aromatisches System ausbilden. Entsprechendes gilt auch für die Rest R⁶, R⁷, R⁸, R⁹, R¹⁰. Hingegen können beispielsweise zwischen dem Rest R⁵ und R⁶ keine kondensierte Systeme ausgebildet werden. Die Reste R⁵ und R⁶ sitzen nicht an dem gleichen Phenylrest.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

In einer Ausführungsform stehen R¹¹ und R¹⁴ für -(C₁-C₁₂)-Alkyl.

In einer Ausführungsform stehen R¹¹ und R¹⁴ für ^{_*ter*}Bu.

In einer Ausführungsform sind R¹², R¹³ ausgewählt aus: -(C₁-C₁₂)-Alkyl, -O-(C₁-C₁₂)-Alkyl.

In einer Ausführungsform stehen R¹² und R¹³ für -OCH₃ oder-*^{ter}*Bu.

In einer Ausführungsform stehen R¹² und R¹³ für -OCH₃.

In einer Ausführungsform sind R¹, R², R³, R⁴ R¹, R², R³, R⁴, R⁵, R⁶, R⁷, R⁸, R⁹, R¹⁰ ausgewählt aus -H, -(C₁-C₁₂)-Alkyl, -(C₄-C₁₂)-Aryl.

In einer Ausführungsform sind R¹, R², R³, R⁴, R⁵, R⁶, R⁷, R⁸, R⁹, R¹⁰ ausgewählt aus -H, -*^{ter}*Bu, -(C₄-C₆)-Aryl.

In einer Ausführungsform weist die Verbindung eine der Strukturen (1) bis (3) auf:

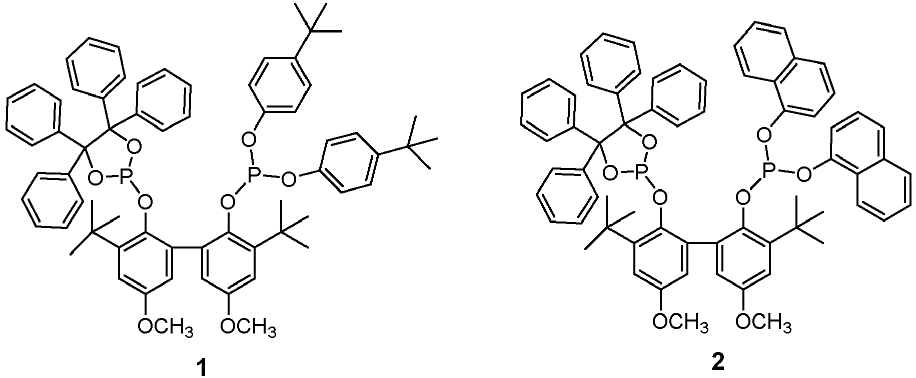

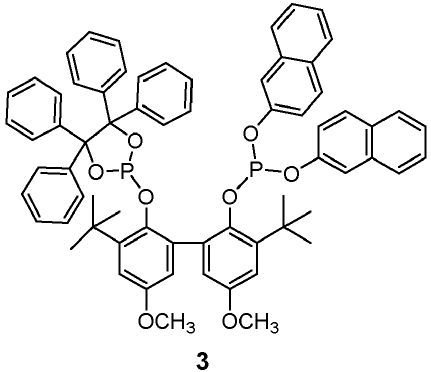

Neben der Verbindung an sich, wird auch ein Verfahren beansprucht, in welchen die Verbindung zum Einsatz kommt.

Verfahren umfassend die Verfahrensschritte:
a) Vorlegen einer ethylenisch ungesättigten Verbindung;
b) Zugabe einer wie zuvor beschriebenen Verbindung
   und einer Substanz, welche Rh umfasst;
c) Zuführen von H₂ und CO,
d) Erwärmen des Reaktionsgemisches aus a) bis c), wobei die ethylenisch ungesättigten Verbindung zu einem Aldehyd umgesetzt wird.

Hierbei können die Verfahrensschritte a), b) und c) in beliebiger Reihenfolge erfolgen. Üblicherweise erfolgt die Zugabe von CO jedoch, nachdem die Reaktionspartner in den Schritten a) und b) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Substanz, welche Rh umfasst, ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens erfolgt das Zuführen von CO in Verfahrensschritt c) mit einem Druck in dem Bereich von 1 bis 6 MPa (10 bis 60 bar).

In einer Variante des Verfahrens erfolgt das Erwärmen des Reaktionsgemisches in Verfahrensschritt d) auf eine Temperatur in dem Bereich von 80 °C bis 160°C.

Im Folgenden soll die Erfindung anhand von Ausführungsbeispielen näher erläutert werden.

### Synthese Bis(4-(tert-butyl)phenyl)(3,3'-di-tert-butyl-5,5'-dimethoxy-2'-((4,4,5,5-tetraphenyl-1,3,2-dioxaphospholan-2-yl)oxy)-n1,1'-biphenyl]-2-yl)phosphit (1):

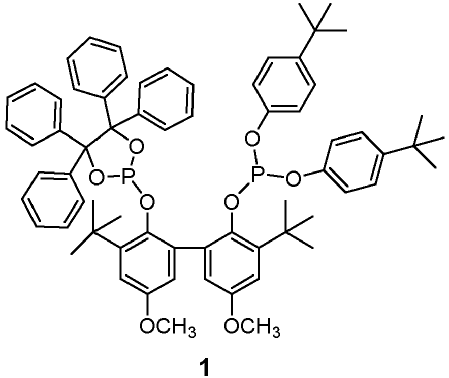

Zu einer Lösung von 2-((3,3'-Di-tert-butyl-2'-((dichlorophosphaneyl)oxy)-5,5'-dimethoxy-[1,1'-biphenyl]-2-yl)oxy)-4,4,5,5-tetraphenyl-1,3,2-dioxaphospholane (0,7254 g; 0,8496 mmol) in 10 ml Toluol wird bei Raumtemperatur tropfenweise eine Mischung von 4-tert.-Butylphenol (0,2808 g; 1,8691 mmol) und Triethylamin (2,39 ml) in 8 ml Toluol gegeben. Man rührt über Nacht, filtriert und engt das Filtrat im Vakuum zur Trockne ein. Der erhaltene Feststoff wird 2 h bei 60°C/ 0,1mbar getrocknet. Ausbeute: 0,869 g (0,8036 mmol, 94%).

Elementaranalyse (ber. für C₆₈H₇₄O₈P₂=1081,273 g/Mol): C = 75,43 (75,53); H = 7,06 (6,90); P = 5,79 (5,73).

ESI-TOF HRMS: *m*/*z*= 1103,4775; [M⁺+Na], ber. *m*/*z*= 1103,4756.

³¹P-NMR (CD₂Cl₂): δ 132,2 (d, *J*_{PP}=49 Hz); 145,3 (d, *J*_{PP}=49 Hz).

¹H-NMR (CD₂Cl₂): δ 1,15 (s, 9H); 1,33 (s, 9H); 1,35 (s, 9H); 1,53 (s, 9H); 3,55 (s, 3H); 3,76 (s, 3H); 6,71-7,43 (m, 32H) ppm.

### Synthese 3,3'-Di-tert-butyl-5,5'-dimethoxy-2'-((4,4,5,5-tetraphenyl-1,3,2-dioxaphospholan-2-yl)oxy)-f1,1'-biphenyl]-2-yldi(naphthalen-1-yl)phosphit (2):

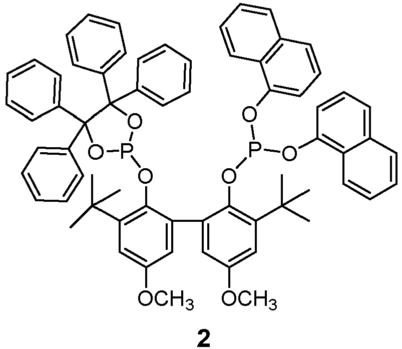

Zu einer Lösung von 2-((3,3'-Di-tert-butyl-2'-((dichlorophosphaneyl)oxy)-5,5'-dimethoxy-[1,1'-biphenyl]-2-yl)oxy)-4,4,5,5-tetraphenyl-1,3,2-dioxaphospholane (0,5622 g; 0,6585 mmol) in 8 ml Toluol wird bei Raumtemperatur tropfenweise eine Mischung von 1-Naphthol (0,2088 g; 1,4487 mmol) und Triethylamin (1,85 ml) in 6 ml Toluol gegeben. Man rührt über Nacht, filtriert und engt das Filtrat im Vakuum zur Trockne ein. Der erhaltene Feststoff wird 2 h bei 60°C/ 0,1mbar getrocknet und anschließend in 6 ml heißem Acetonitril aufgenommen. Der nach Lagerung der Lösung bei -29 °C erhaltene Feststoff wird abgetrennt, mit wenig kaltem Acetonitril gewaschen und getrocknet. Ausbeute: 0,480 g (0,449 mmol, 68%). Elementaranalyse (ber. für C₆₈H₆₂O₈P₂₌1069, 178 g/Mol): C = 76,36 (76,39); H = 5,95 (5,85); P = 5,67 (5,79).

ESI-TOF HRMS: *m*/*z*=1091,3801; [M⁺+Na], ber. *m*/*z*=1091,3817.

³¹P-NMR (CD₂Cl₂): δ 135,5 (d, *J*_{PP}=30 Hz); 146,2 (d, *J*_{PP}=30 Hz) ppm.

¹H-NMR (CD₂Cl₂): δ 1,14 (s, 9H); 1,63 (s, 9H); 2,94 (s, 3H); 3,87 (s, 3H); 6,33 (d, ⁴*J*_{HH}=3,1 Hz; 1H); 6,74 (d, ⁴*J*_{HH}=3,1 Hz; 1H); 6,89-7,84 (m, 35 H); 8,12 (m, 1H) ppm.

### Synthese 3,3'-di-tert-butyl-5,5'-dimethoxy-2'-((4,4,5,5-tetraphenyl-1,3,2-dioxaphospholan-2-yl)oxy)-[1,1'-biphenyl]-2-yl di(naphthalen-2-yl)phosphit (3):

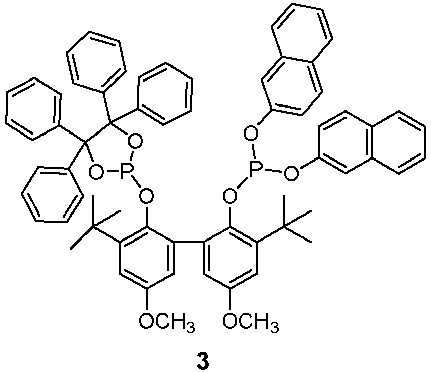

Zu einer Lösung von 2-((3,3'-Di-tert-butyl-2'-((dichlorophosphaneyl)oxy)-5,5'-dimethoxy-[1,1'-biphenyl]-2-yl)oxy)-4,4,5,5-tetraphenyl-1,3,2-dioxaphospholan (0,5971 g; 0,6993 mmol) in 8 ml Toluol wird bei Raumtemperatur tropfenweise eine Mischung von 2-Naphthol (0,2218 g; 1,5386 mmol) und Triethylamin (1,96 ml) in 6 ml Toluol gegeben. Man rührt über Nacht, filtriert und engt das Filtrat im Vakuum zur Trockne ein. Der erhaltene Feststoff wird 2 h bei 60°C/ 0,1 mbar getrocknet und anschließend in 6,5 ml heißem Acetonitril aufgenommen. Der nach Lagerung der Lösung bei -29 °C erhaltene Feststoff wird abgetrennt, mit wenig kaltem Acetonitril gewaschen und getrocknet. Ausbeute: 0,470 g (0,439 mmol, 63%). Elementaranalyse (ber. für C₆₈H₆₂O₈P₂₌1069,178 g/Mol): C = 76,18 (76,39); H = 5,88 (5,85); P = 5,74 (5,79).

ESI-TOF HRMS: *m*/*z*=1091,3811; [M⁺+Na], ber. *m*/*z*=1091,3817.

³¹P-NMR (CD₂Cl₂): δ 131,8 (d, *J*_{PP}=56 Hz); 145,0 (d, *J*_{PP}=56 Hz) ppm.

¹H-NMR (CD₂Cl₂): δ 1,15 (s, 9H); 1,58 (s, 9H); 3,48 (s, 3H); 3,77 (s, 3H); 6,78 (m, 1H); 6,83 (m, 1H); 6,86 (m, 1H); 6,93-7,03 (m, 8H); 7,07-7,23 (m, 11); 7,35-7,52 (m, 10 H); 7,66 (m, 1H); 7,72-7,85 (m, 5 H) ppm.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD = 1,5-Cyclooctadien), zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Die Reaktion wurde mit den erfindungsgemäßen Verbindungen (1) bis (3), sowie mit dem Vergleichsliganden (D-1) durchgeführt.

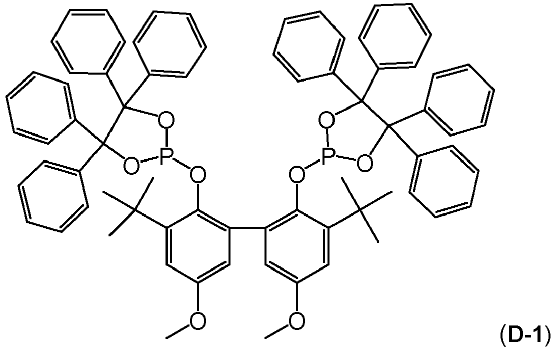

### Reaktionsbedingungen:

Olefin: 2-Penten, Solvens: Toluol, Masseanteil Rhodium: 100 ppm, p: 20 bar, T: 120 °C, t: 4 h, Verhältnis Rh : Ligand = 1 : 2.

Die Ergebnisse sind in der nachfolgenden Tabelle zusammengestellt:

| Ligand | Ausbeute Aldehyd [%] |
|---|---|
| **1*** | 46 |
| **2*** | 45 |
| **3*** | 50 |
| **D-1** | 14 |

| | |
|---|---|
| *erfindungsgemäße Verbindung | |

Wie die Versuchsergebnisse zeigen, wird die Aufgabe durch die erfindungsgemäßen Verbindungen gelöst.

## Patentansprüche

1. Verbindung gemäß Formel (I): 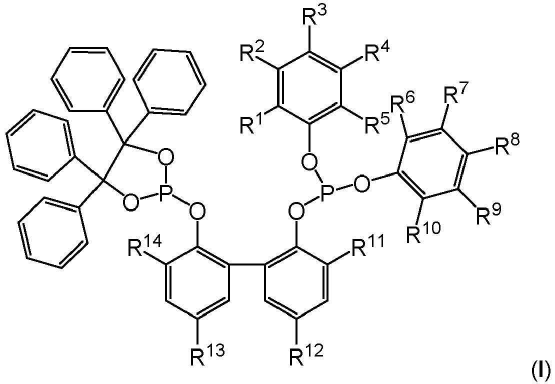 wobei
R¹, R², R³, R⁴, R⁵, R⁶, R⁷, R⁸, R⁹, R¹⁰, R¹¹, R¹², R¹³, R¹⁴ jeweils unabhängig voneinander ausgewählt sind aus: -H, -(C₁-C₁₂)-Alkyl, -O-(C₁-C₁₂)-Alkyl, -(C₄-C₁₂)-Aryl und die Reste R¹, R², R³, R⁴, R⁵, sowie die Reste R⁶, R⁷, R⁸, R⁹, R¹⁰ untereinander kondensierte Systeme ausbilden können.

2. Verbindung nach Anspruch 1,
wobei R¹¹ und R¹⁴ für -(C₁-C₁₂)-Alkyl stehen.

3. Verbindung nach einem der Ansprüche 1 oder 2,
wobei R¹¹ und R¹⁴ für -*^{ter}*Bu stehen.

4. Verbindung nach einem der Ansprüche 1 bis 3,
wobei R¹², R¹³ ausgewählt sind aus: -(C₁-C₁₂)-Alkyl, -O-(C₁-C₁₂)-Alkyl.

5. Verbindung nach einem der Ansprüche 1 bis 4,
wobei R¹² und R¹³ für -OCH₃ oder-*^{ter}*Bu stehen.

6. Verbindung nach einem der Ansprüche 1 bis 5,
wobei R¹, R², R³, R⁴, R⁵, R⁶, R⁷, R⁸, R⁹, R¹⁰ ausgewählt sind aus -H, -(C₁-C₁₂)-Alkyl, -(C₄-C₁₂)-Aryl.

7. Verbindung nach einem der Ansprüche 1 bis 8,
wobei R¹, R², R³, R⁴, R⁵, R⁶, R⁷, R⁸, R⁹, R¹⁰ ausgewählt sind aus -H, -*^{ter}*Bu, -(C₄-C₆)-Aryl.

8. Verbindung nach einem der Ansprüche 1 bis 7,
wobei die Verbindung eine der Strukturen (1) bis (3) aufweist:

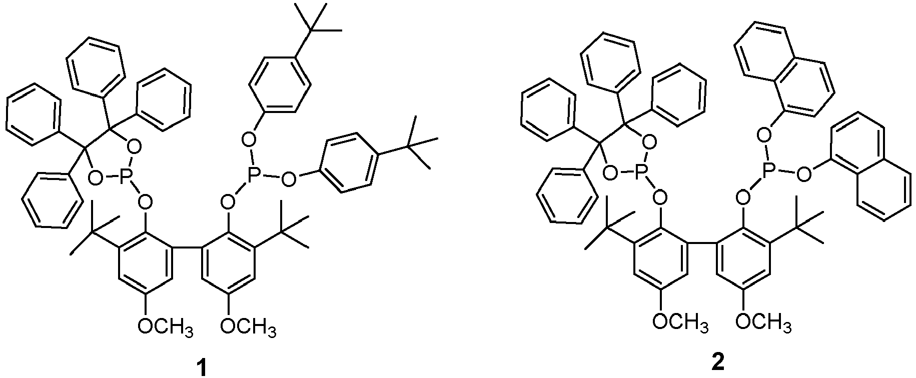

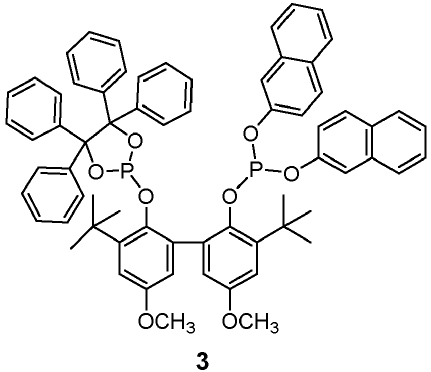

9. Verfahren umfassend die Verfahrensschritte:
a) Vorlegen einer ethylenisch ungesättigten Verbindung;
b) Zugabe einer Verbindung gemäß einem der Ansprüche 1 bis 8 und einer Substanz, welche Rh umfasst;
c) Zuführen von H₂ und CO,
d) Erwärmen des Reaktionsgemisches aus a) bis c), wobei das Olefin zu einem Aldehyd umgesetzt wird.

10. Verfahren nach Anspruch 9,
wobei die ethylenisch ungesättigte Verbindung in Verfahrensschritt a) ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

11. Verfahren nach einem der Ansprüche 9 oder 10,
wobei die Substanz, welche Rh umfasst, ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

12. Verfahren nach einem der Ansprüche 9 bis 11,
wobei das Zuführen von CO in Verfahrensschritt c) mit einem Druck in dem Bereich von 1 bis 6 MPa (10 bis 60 bar) erfolgt.

13. Verfahren nach einem der Ansprüche 9 bis 12,
wobei das Erwärmen des Reaktionsgemisches in Verfahrensschritt d) auf eine Temperatur in dem Bereich von 80 °C bis 160 °C erfolgt.

## Claims

1. Compound of formula (I): 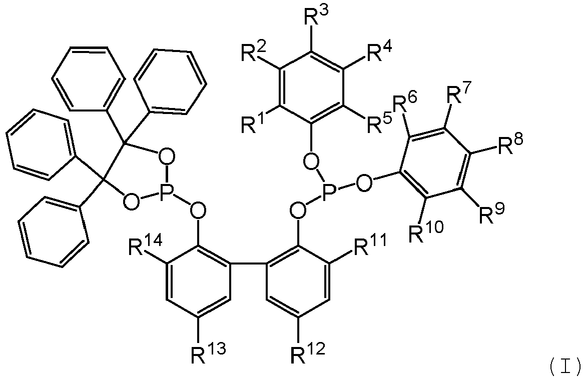 wherein
R¹, R², R³, R⁴, R⁵, R⁶, R⁷, R⁸, R⁹, R¹⁰, R¹¹, R¹², R¹³, R¹⁴ are each independently selected from: -H, -(C₁-C₁₂)-alkyl, - O-(C₁-C₁₂)-alkyl, -(C₄-C₁₂)-aryl, and the radicals R¹, R², R3, R⁴, R⁵, and also the radicals R⁶, R⁷, R⁸, R⁹, R¹⁰, may form fused systems with one another.

2. Compound according to Claim 1,
wherein R¹¹ and R¹⁴ are -(C₁-C₁₂)-alkyl.

3. Compound according to either of Claims 1 and 2,
wherein R¹¹ and R¹⁴ are -*^{tert}*Bu.

4. Compound according to any of Claims 1 to 3,
wherein R¹², R¹³ are selected from: -(C₁-C₁₂)-alkyl, -O-(C₁-C₁₂)-alkyl.

5. Compound according to any of Claims 1 to 4,
wherein R¹² and R¹³ are -OCH₃ or -*^{tert}*Bu.

6. Compound according to any of Claims 1 to 5,
wherein R¹, R², R³, R⁴, R⁵, R⁶, R⁷, R⁸, R⁹, R¹⁰ are selected from -H, -(C₁-C₁₂)-alkyl, -(C₄-C₁₂)-aryl.

7. Compound according to any of Claims 1 to 8,
wherein R¹, R², R³, R⁴, R⁵, R⁶, R⁷, R⁸, R⁹, R¹⁰ are selected from -H, -*^{tert}*Bu, -(C₄-C₆)-aryl.

8. Compound according to any of Claims 1 to 7,
wherein the compound has one of the structures (1) to (3) :

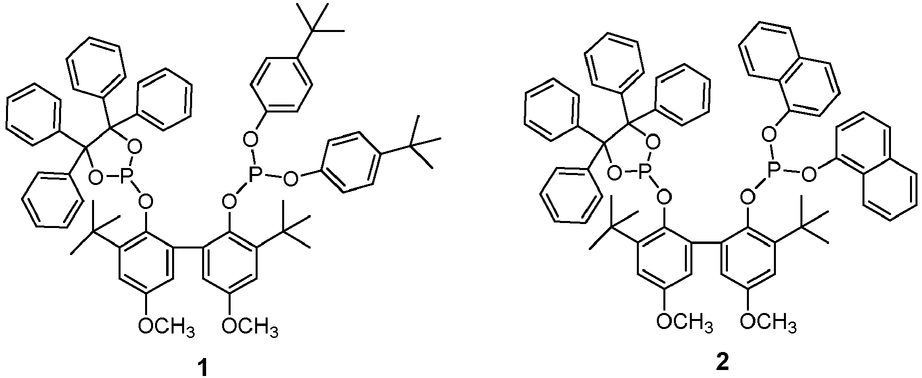

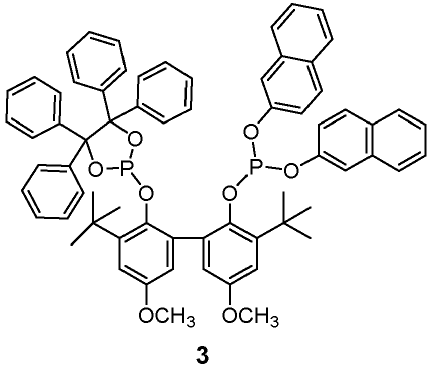

9. Process comprising the process steps of:
a) initially charging an ethylenically unsaturated compound;
b) adding a compound according to any of Claims 1 to 8
and a substance comprising Rh;
c) feeding in H₂ and CO,
d) heating the reaction mixture from a) to c), with conversion of the olefin to an aldehyde.

10. Process according to Claim 9,
wherein the ethylenically unsaturated compound in process step a) is selected from: ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, or mixtures thereof.

11. Process according to either of Claims 9 and 10, wherein the substance comprising Rh is selected from: Rh(acac)(CO)₂, [(acac)Rh(COD)] (Umicore, acac = acetylacetonate anion; COD = 1,5-cyclooctadiene), Rh₄CO₁₂.

12. Process according to any of Claims 9 to 11,
wherein CO is fed in in process step c) at a pressure in the range from 1 to 6 MPa (10 to 60 bar).

13. Process according to any of Claims 9 to 12,
wherein the reaction mixture is heated in process step d) to a temperature in the range from 80°C to 160°C.

## Revendications

1. Composé selon la formule (I) : 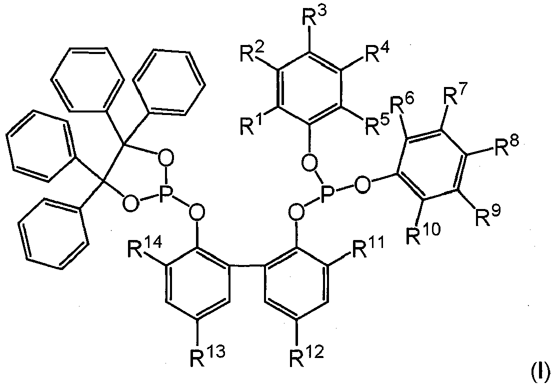 dans laquelle
R¹, R², R³, R⁴, R⁵, R⁶, R⁷, R⁸, R⁹, R¹⁰, R¹¹, R¹², R¹³, R¹⁴ sont choisis, à chaque fois indépendamment les uns des autres, parmi : -H, -(C₁-C₁₂)-alkyle, -O-(C₁-C₁₂)-alkyle, -(C₄₋C₁₂)-aryle et les radicaux R¹, R², R³, R⁴, R⁵ ainsi que les radicaux R⁶, R⁷, R⁸, R⁹, R¹⁰ peuvent former des systèmes mutuellement condensés.

2. Composé selon la revendication 1, R¹¹ et R¹⁴ représentant -(C₁-C₁₂)-alkyle.

3. Composé selon l'une quelconque des revendications 1 ou 2, R¹¹ et R¹⁴ représentant -^{ter}Bu.

4. Composé selon l'une quelconque des revendications 1 à 3, R¹², R¹³ étant choisis parmi : -(C₁-C₁₂)-alkyle, -O-(C₁-C₁₂)-alkyle.

5. Composé selon l'une quelconque des revendications 1 à 4, R¹² et R¹³ représentant -OCH₃ ou -^{ter}Bu.

6. Composé selon l'une quelconque des revendications 1 à 5, R¹, R², R³, R⁴, R⁵, R⁶, R⁷, R⁸, R⁹, R¹⁰ étant choisis parmi -H, -(C₁-C₁₂)-alkyle, -(C₄-C₁₂)-aryle.

7. Composé selon l'une quelconque des revendications 1 à 8, R¹, R², R³, R⁴, R⁵, R⁶, R⁷, R⁸, R⁹, R¹⁰ étant choisis parmi -H, -^{ter}Bu, -(C₄₋C₆) -aryle.

8. Composé selon l'une quelconque des revendications 1 à 7, le composé présentant l'une des structures (1) à (3) :

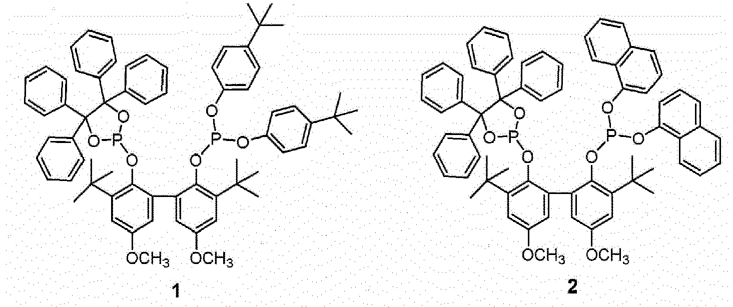

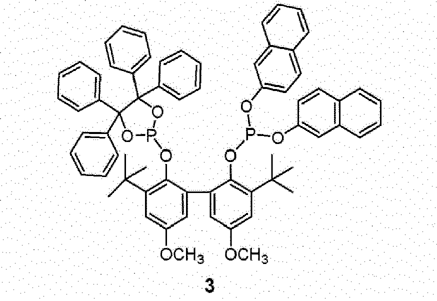

9. Procédé comprenant les étapes de procédé :
a) disposition préalable d'un composé éthyléniquement insaturé ;
b) ajout d'un composé selon l'une quelconque des revendications 1 à 8 et d'une substance qui comprend Rh ;
c) introduction de H₂ et de CO ;
d) chauffage du mélange réactionnel de a) à c), l'oléfine étant transformée en aldéhyde.

10. Procédé selon la revendication 9, le composé éthyléniquement insaturé dans l'étape de procédé a) étant choisi parmi : l'éthylène, le propène, le 1-butène, le cis-2-butène et/ou le trans-2-butène, l'iso-butène, le 1,3-butadiène, le 1-pentène, le cis-2-pentène et/ou le trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

11. Procédé selon l'une quelconque des revendications 9 à 10, la substance comprenant Rh étant choisie parmi : Rh(acac)(CO)₂, [(acac)Rh(COD)] (Umicore, acac = anion acétylacétonate ; COD = 1,5-cyclooctadiène), Rh₄CO₁₂.

12. Procédé selon l'une quelconque des revendications 9 à 11, l'introduction de CO dans l'étape de procédé c) ayant lieu à une pression dans la plage de 1 à 6 MPa (10 à 60 bars).

13. Procédé selon l'une quelconque des revendications 9 à 12, le chauffage du mélange réactionnel dans l'étape de procédé d) ayant lieu à une température dans la plage de 80°C à 160°C.
